# EUROPEAN PATENT SPECIFICATION

(11) **EP 0 994 740 B1**
(45) Date of publication and mention of the grant of the patent: **30.07.2003**
(21) Application number: 99913870.4
(22) Date of filing: 12.03.1999
(51) Int. Cl.: A61M 5/00, A61B 17/34

(54) **CONE-SHAPED UNIVERSAL SEAL HAVING NONUNIFORM SURFACE AND SEAL ASSEMBLY INCLUDING SAME**
KEGELFÖRMIGE UNIVERSELLE ABDICHTUNG, INSBESONDERE FÜR TROKARBESTECK
JOINT D'ETANCHEITE UNIVERSEL CONIQUE

(30) Priority: 13.04.1998 US 59195
(43) Date of publication of application: 26.04.2000
(73) Proprietor: Applied Medical Resources Corporation, Rancho Santa Margarita, CA 92688 (US)
(72) Inventor: TO, John, T., Sunnyvale, CA 94086 (US); GRESL, Charles, San Francisco, CA 94131 (US); KRAMER, Thomas, A., San Carlos, CA 94070 (US); VARGAS, Jaimes, Palo Alto, CA 94303 (US)
(74) Representative: Enskat, Michael Antony Frank
(86) International application number: US9905486
(87) International publication number: WO99052577

(56) References cited:
- WO-A-97/47248
- US-A- 5 342 315
- US-A- 5 385 553
- US-A- 5 411 483
- US-A- 5 545 142
- US-A- 5 628 732

## Description

### Field of the Invention

The invention pertains to a universal instrument seal assembly configured to be removably snapped onto a cannula-seal system including a valve door assembly that is removably snapped onto a cannula. Both universal and valve door assemblies are intended for use during a single medical procedure (such as a laparoscopic procedure) and the cannula is designed for multiple use (each time with a different valve door assembly) during multiple medical procedures. The universal seal assembly includes a cone-shaped element having a nonuniform (e.g., ribbed or dimpled) surface surrounding an orifice. The cone-shaped element is shaped to direct a medical instrument toward the orifice so that when the instrument has been inserted through the orifice, the cone-shaped element provides a fluid seal around the instrument.

### Background of the Invention

Many medical procedures require use of a cannula through which one or more medical instruments are inserted into a patient and then removed from the patient.

For example, in a variety of laparoscopic procedures (including laparoscopic hernia repair), a cannula is positioned with its distal end inside the patient and its proximal end outside the patient, and one or more medical instruments are inserted through the cannula into the patient. For example, each of a sequence of instruments (including an endoscope) can be inserted through the cannula into the patient and then withdrawn (in the opposite direction) out from the patient and cannula.

During many such procedures, it is necessary to maintain an insufflated working space within the patient (by maintaining insufflating gas at sufficiently high pressure in the working space) while the distal end of the cannula extends into the working space. For use during these procedures, the cannula must be provided with a seal or seals for preventing undesired fluid escape from within the patient out through the cannula. The term "fluid" is used herein to denote either a gas or a liquid. One such seal (denoted herein as a "fluid" seal) prevents fluid escape from the cannula when no instrument occupies the cannula's channel. A fluid seal is implemented as a flapper valve, duckbill valve, or other valve, which is biased in a closed position at times when no instrument occupies the cannula's channel to provide a fluid seal preventing fluid flow through the channel at such times. When the distal end of an instrument is inserted into the channel and the instrument is advanced through the channel toward the patient, the instrument opens the fluid seal (e.g., by displacing the flexible slits of a duckbill valve or displacing the trap door of a flapper valve).

Typically also, an additional seal (sometimes denoted herein as an "instrument seal") is employed in a cannula to provide a fluid seal around the instrument's outer periphery, to prevent fluid flow through the space between the instrument and the wall of the channel. An instrument seal adapted for sealing around an instrument having any of a range of diameters, typically despite lateral translation (translation perpendicular to the cannula's longitudinal axis) and/or pivoting (relative to the cannula's longitudinal axis) of the instrument extending through the seal, the instrument seal is sometimes known as a "universal" seal.

U.S. Patent 5,545,142, issued August 13, 1996, discloses a seal assembly including an elastomeric universal seal 134 mounted, in a rigid housing 116 of a trocar device 100, to a rigid guide/retainer element 142 and a rigid portion of a flap valve assembly 136. The universal seal has a planar inner portion 144 around an orifice 148, and an outer portion 146 surrounding inner portion 144. Radially oriented ribs 158 are provided on inner portion 144 and a portion of outer portion 146 to reduce surface contact between the seal surface and an instrument being inserted through orifice 148. The flap valve assembly is mounted within the trocar housing to provide a fluid seal when no instrument extends through seal 134. When an instrument is pushed toward seal 134, guide/retainer element 142 guides the instrument's tip toward orifice 148. If element 142 were omitted, the planar shape of inner portion 144 would undesirably cause seal 134 to be vulnerable to tearing or puncture by an instrument (especially a sharp-tipped instrument) being inserted in a distal direction, but off-axis (i.e., with the instrument's longitudinal axis offset from the seal's central axis which extends through the center of orifice 148), into engagement with portion 144. Also, since seal 134 is mounted within a rigid housing, it is neither easily cleanable after it has been used nor easily removable for replacement if damaged.

U.S. Patent 5,628,732, issued May 13, 1997, discloses a universal seal according to the preamble of claim 1 mounted within a rigid frame. The seal has a central aperture surrounded by a generally conical, laminated element. The laminated element comprises a generally conical layer of relatively soft material, and a layer of relatively hard material overlying (and co-molded with) a patterned region on the relatively soft layer. Since the seal includes two co-molded pieces of material, it will be too stiff (insufficiently compliant) for many applications and is expensive and complicated to manufacture. The complicated design of the rigid frame in which the seal is mounted also increases the expense and complexity of manufacture of the apparatus. Also, since the seal is mounted in a rigid frame, it is neither easily cleanable after it has been used nor easily removable for replacement if damaged.

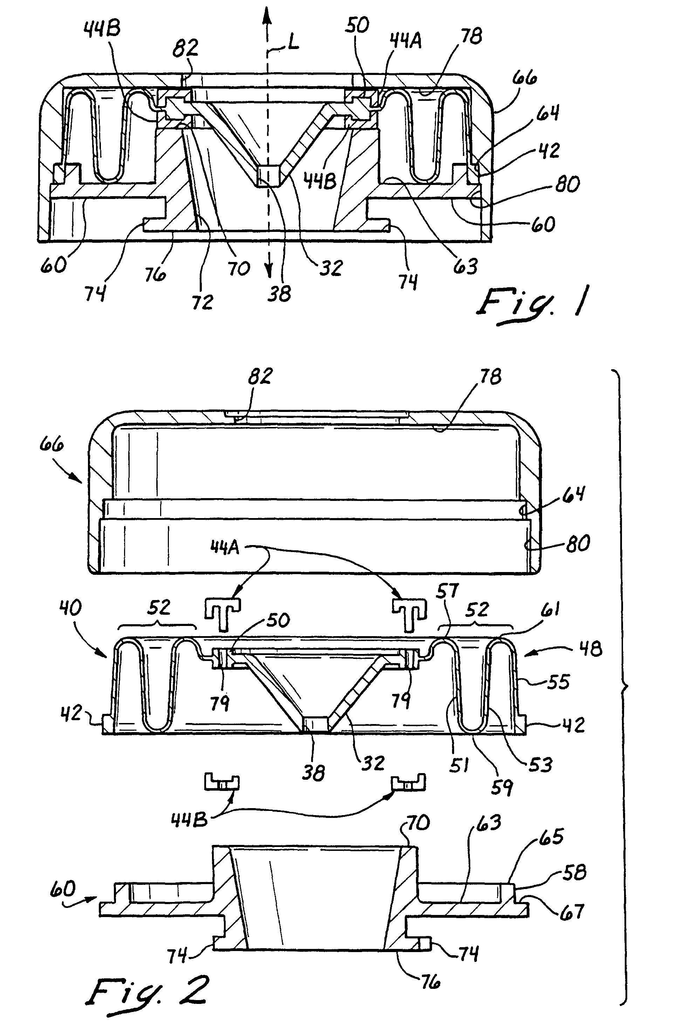

U.S. Patent 5,549,565 discloses a disposable valve assembly including a flapper valve and a universal seal (with annular bumps 263a and 263b as shown in Fig. 2p thereof) against which the flapper valve is biased, and another universal seal (with annular rings 163a and 163b as shown in Fig. 2e thereof) for use with a slit valve. However, neither seal has a conical surface for guiding an instrument toward the flapper valve or slit valve.

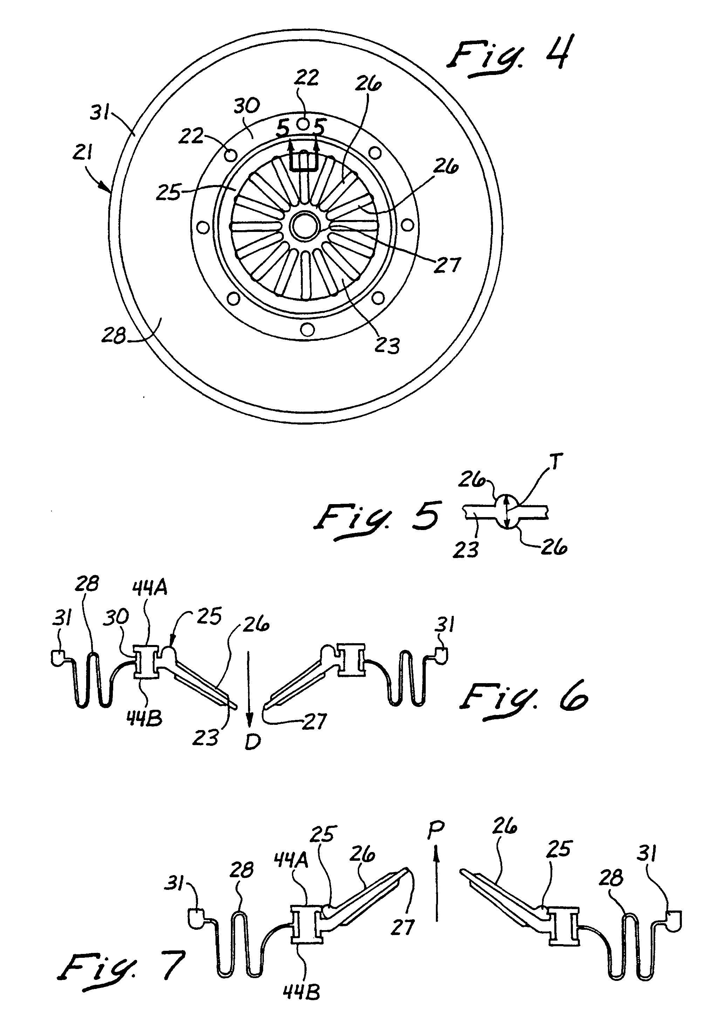

U.S. Patent 5,385,552, issued January 31, 1995, discloses a seal assembly (as shown in Fig. 4 thereof) including a generally conical instrument seal 136 and an elastomeric fluid seal, both mounted in a rigid housing. The instrument seal has concentric annular ribs on its conical surface, which are said to reduce sliding friction of tubes passing through the instrument seal.

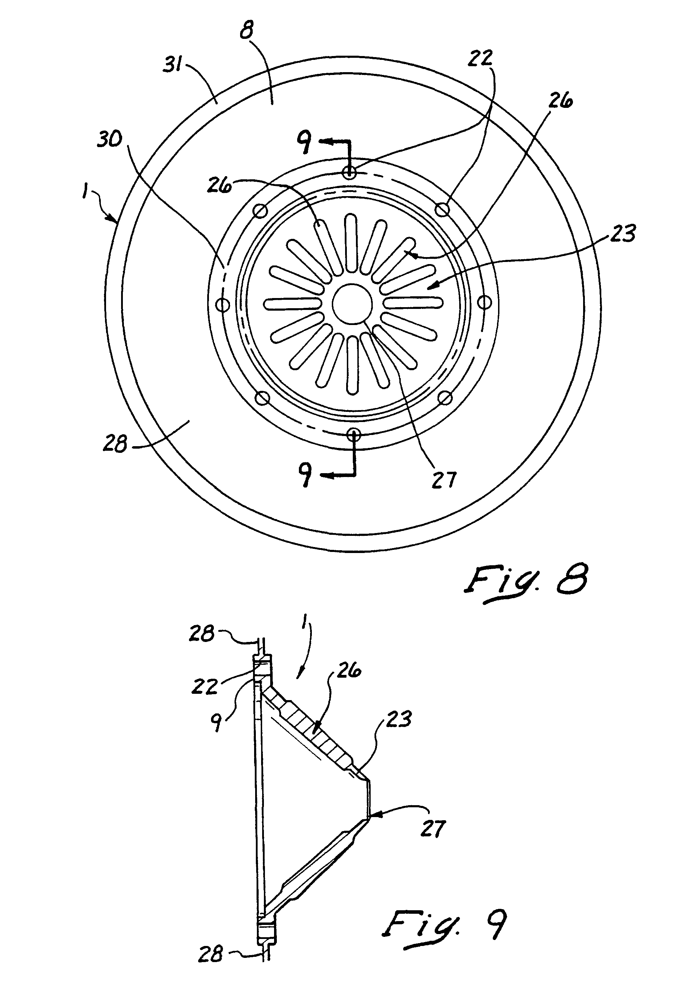

U.S. Patent 5,411,483, issued May 2, 1995 and assigned to the assignee of the present invention, discloses a universal seal assembly including a smooth, conical instrument seal mounted in a rigid housing (as shown in Figs. 5A and 5B thereof). The housing can be coupled to another rigid housing in which a flapper valve is mounted.

The universal seal assembly of U.S. Patent 5,411,483 will be described with reference to Figures 1 and 2 of the present disclosure. The universal seal assembly comprises seal molding 40 (typically molded from elastomeric material), base 60 and cap 66 (both typically formed of rigid plastic), and stabilizing ring halves 44A and 44B. Molding 40 comprises conical instrument seal 32 (the central portion of molding 40), stabilizing ring anchor 50 surrounding conical seal 32, corrugated annular portion 52 surrounding anchor 50, and anchoring ring 42 surrounding corrugated portion 52. Ring halves 44A and 44B are snapped together to define a ring which encloses ring anchor 50 of seal 32, and base 60 and cap 66 are assembled to define a rigid housing surrounding molding 40 and ring halves 44A and 44B. The ring defined by halves 44A and 44B is configured to constrain motion of seal 32 perpendicular to longitudinal axis L, when seal 32 has been mounted as shown within the assembled housing. Cap 66 has an orifice 82 through its center, through which an instrument can be inserted. Conical seal 32 has an orifice 38 (also referred to as an instrument port) through its center, and functions to guide the tip of an instrument radially inward toward orifice 38 and in the distal direction (downward along longitudinal axis L shown in Fig. 1) toward orifice 38.

Molding 40 is molded so that corrugated portion 52 biases orifice 38 to be centered about longitudinal axis L when the assembly has been assembled. Orifice 38 has freedom to translate in a plane perpendicular to the longitudinal axis in response to radial displacement of the orifice's rim (by an instrument extending through the orifice), since corrugated portion 52 is compliant in the radial direction and flexes in response to the force exerted on seal 32 by the instrument.

The distally-extending conical shape of instrument seal 32 reduces the ability of an instrument with a sharp (e.g., pronged) distal end to penetrate the instrument seal by generating a lateral component from the force between the instrument and the instrument seal. The lateral force component laterally displaces the material surrounding instrument port 38 and enables the pronged end of the instrument to enter the instrument port. In other words, the pronged end of the instrument is guided along the conical surface of seal 32 and enters port 38. However, the design of conventional instrument seal 32 (which is improved in accordance with the present invention) is subject to problems of instrument drag (due to friction) on the seal, and inversion (when an instrument is removed from the instrument port in a proximal direction).

The thickness of conical instrument seal 32 of Figs. 1 and 2 is typically in the range of 0.02" to 0.06" (0.51 - 1.52 mm), with a preferred thickness of 0.03" (0.76 mm).

The preferred material of seal molding 40 is silicone rubber (which has excellent ability to recover after deformation, but less ability to resist penetration). Polyurethane is an alternative material for the seal molding. The penetration resistance of polyurethane is superior to that of silicone rubber, but its ability to recover after deformation is inferior. Typically, instrument seal 32 of molding 40 is coated with an anti-friction coating.

The lateral compliance of corrugated zone 52 is increased by molding it so that it has a series of substantially vertical elements 51, 53, and 55, interconnected by substantially semicircular sections 57, 59, and 61. Its lateral compliance is further increased by increasing the length of vertical elements 51, 53, and 55. The material of corrugated zone 52 bends and/or buckles to accommodate the lateral movement of instrument seal 32 (with stabilizing anchor 50, and ring halves 44A and 44B mounted to ring anchor 50) relative to fixed anchoring ring 42. The preferred thickness of corrugated zone 52 is 0.01" (0.25 mm), in typical implementations.

Base 60 and cap 66 are shaped and sized to accommodate corrugated zone 52 between face 78 of cap 66 and pocket 63 of base 60, and to allow instrument seal 32 to move laterally. Face 70 of base 60, on which ring halves 44A and 44B slide during lateral motion of seal 32, occupies a relatively small fraction of the area of base 60.

Lugs 74 and sealing face 76 at the distal end of base 60 are provided to attach the universal seal assembly to the proximal end of the housing of a trocar tube (not shown), with lugs 74 engaged with grooves in the trocar tube housing, and a fluid seal engaged with sealing face 76.

Cap 66 is formed with internal circumferential steps 64 and 80. Anchoring ring 42 of molding 40 is held in the annular groove formed between outer face 58 of outer wall 65 of base 60, annular part 67 of base 60 (extending radially outside wall 65), and step 64 of cap 66. The annular groove locates and forms a gas-tight seal with anchoring ring 42. Part 67 of base 60 fits into step 80 and defines the relative axial location of the base and cap.

A stabilizing ring for molding 40 is formed by stabilizing ring halves 44A and 44B. Each stabilizing ring half is an annular plastic ring half molding. Several (e.g., eight) equally-spaced pin holes 79 are formed through stabilizing ring anchor 50 of molding 40. Ring halves 44A and 44B are attached to molding 40 by inserting the pins of ring halves 44A through pin holes 79 in molding 40, and pressed the pins into corresponding pin holes in ring halves 44B.

It has been suggested to form seal 32 with flutes extending from instrument port 38 to stabilizing ring anchor 50, or with a textured, marbled, or matt finish, or to attach cut-resistant elements (such as overlapping polyurethane scales) to the instrument-contacting surface of seal 32 (or to form seal 32 with such cut-resistant elements) to increase the penetration resistance of the surface and reduce friction between seal 32 and an instrument being inserted in a generally distal direction through port 38.

It had not been known until the present invention how to design a universal seal assembly (having a generally conical, elastomeric seal surrounding an orifice, for providing a fluid seal around the orifice when an instrument having any of a range of diameters extends through the orifice) to be conveniently and removably attachable to a cannula-seal system, where the cannula-seal system includes a disposable seal assembly removably snapped onto a cannula (e.g., where both the universal and disposable seal assemblies are intended for use during a single medical procedure and the cannula is designed for multiple use, each time with a different disposable seal assembly, during multiple medical procedures). Nor had it been known to form the generally conical elastomeric seal surface of such a universal seal assembly to be nonuniform (e.g., ribbed) to provide an acceptably low level of friction on an instrument being inserted distally toward or through the orifice (especially an instrument whose diameter is the largest in the range of diameters for use with the universal seal assembly) and also to rebound efficiently to its original configuration after being inverted by an instrument being withdrawn proximally from the orifice.

According to the present invention there is provided a universal seal assembly for use with a valve door assembly having an elastomeric body, the universal seal assembly including: a housing through which a channel extends, the housing having a mounting portion configured for attaching the housing to the elastomeric body of the valve door assembly; and an elastomeric universal seal molding having a periphery, a generally conical instrument guide defining an instrument port, and a flexible corrugated portion between the periphery and the instrument guide, the molding being mounted in the housing with the periphery fixedly attached to the housing and the instrument guide free to translate relative to the housing so that the instrument port has variable position in the channel; a ring attached to the molding between the instrument guide and the corrugated portion, the ring being configured to constrain motion of the instrument guide in a direction perpendicular to the axis of the channel characterised in that the molding has a portion which defines a bumper extending around the instrument guide and protrudes toward the ring from the instrument guide, the bumper being sized and shaped to cause the universal seal molding to rebound efficiently off the ring to its original conical configuration after being inverted in response to exertion of force thereon.

A universal seal assembly, embodying the present invention, will now be described, by way of example, with reference to the accompanying diagrammatic drawings, in which:
Figure 1 is a cross-sectional view of a conventional universal seal assembly with a conical instrument seal;
Figure 2 is an exploded cross-sectional view of the seal assembly of Fig. 1;
Figure 3 is a side elevational view of a reusable cannula, a valve door assembly snapped onto the cannula, and a universal seal assembly (designed in accordance with a preferred embodiment of the invention) snapped onto the valve door assembly;
Figure 4 is a top elevational view of a preferred embodiment of the universal instrument seal of the universal seal assembly of Fig. 3;
Figure 5 is a side cross-sectional view, taken along line 5-5 of Fig. 4 of a portion of the instrument seal of Fig. 4;
Figure 6 is a side cross-sectional view of the instrument seal of Fig. 4;
Figure 7 is a side cross-sectional view of the instrument seal of Fig. 4, in an inverted configuration.
Figure 8 is a top elevational view of an universal instrument seal.
Figure 9 is a side cross-sectional view, taken along line 9-9 of Fig. 8, of a portion of the instrument seal of Fig. 8.
Figure 10 is a top elevational view of another seal molding.
Figure 11 is a side cross-sectional view of a portion of one implementation of the instrument seal molding of Fig. 10.
Figure 12 is a side cross-sectional view of a portion of another implementation of the instrument seal molding of Fig. 10.
Figure 13 is a side elevational view of a valve door assembly (onto which a preferred embodiment of the invention can be snapped) and four reusable cannulae (each cannula usable with the seal).
Figure 14 is a side cross-sectional view of valve door assembly 202 of Fig. 13.
Figure 15 is a simplified, side elevational view of an alternative embodiment of the inventive universal seal assembly snapped onto a valve door assembly (the valve door assembly is removably mounted to a reusable cannula).
Figure 16 is a simplified, side elevational view of another embodiment of the inventive universal seal assembly snapped onto a valve door assembly (the valve door assembly is removably mounted to a reusable cannula).

### Detailed Description of the Preferred Embodiments

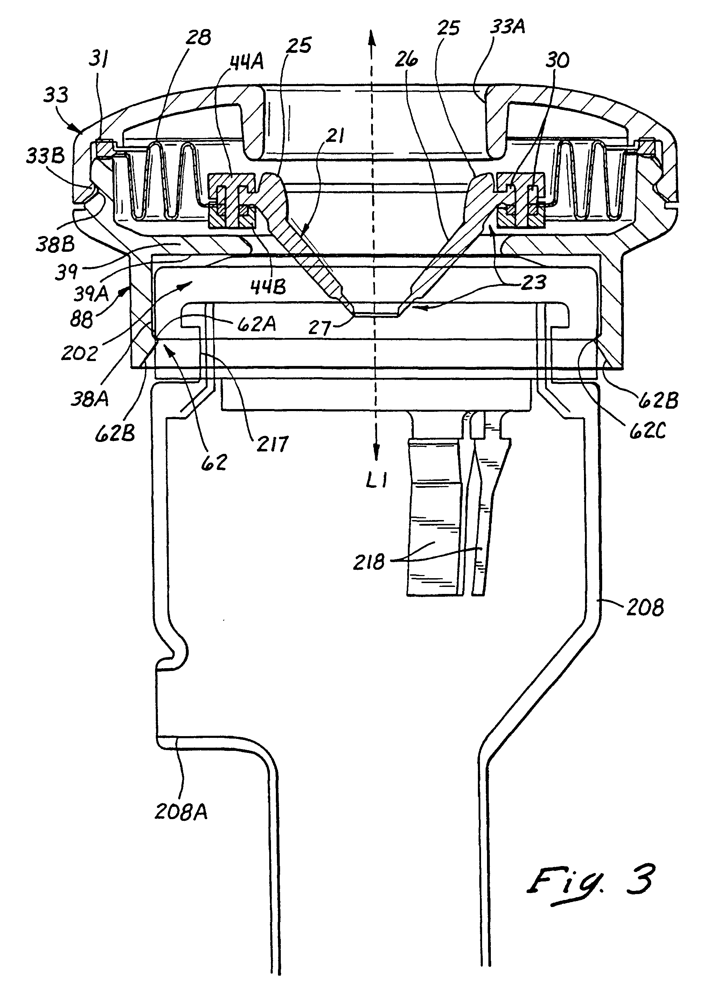

A preferred embodiment of the invention will be described with reference to Figures 3-7. A

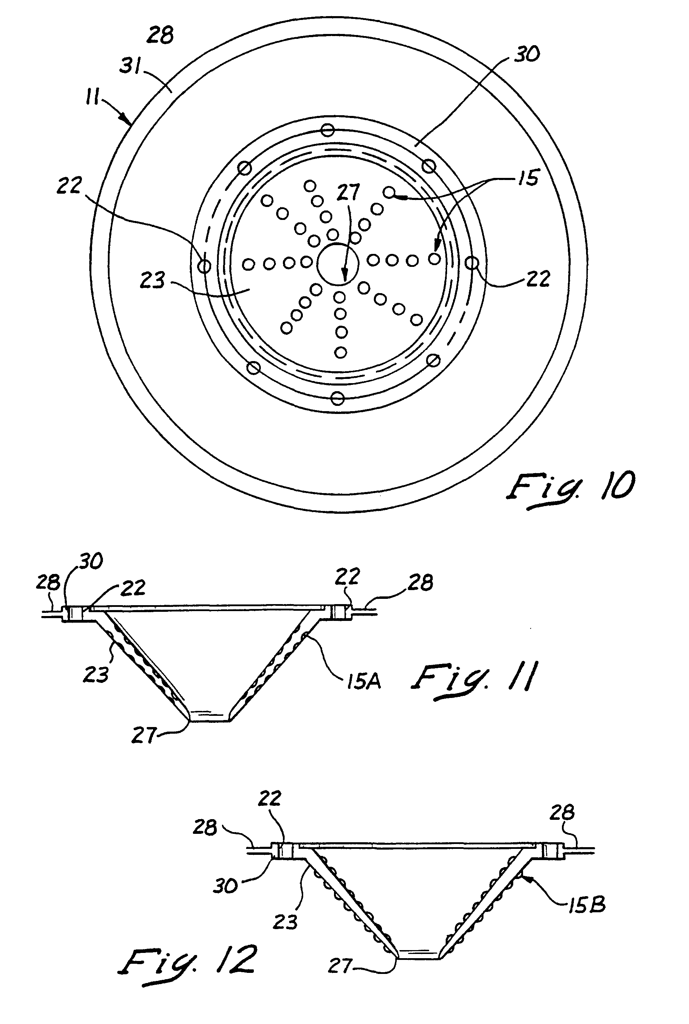

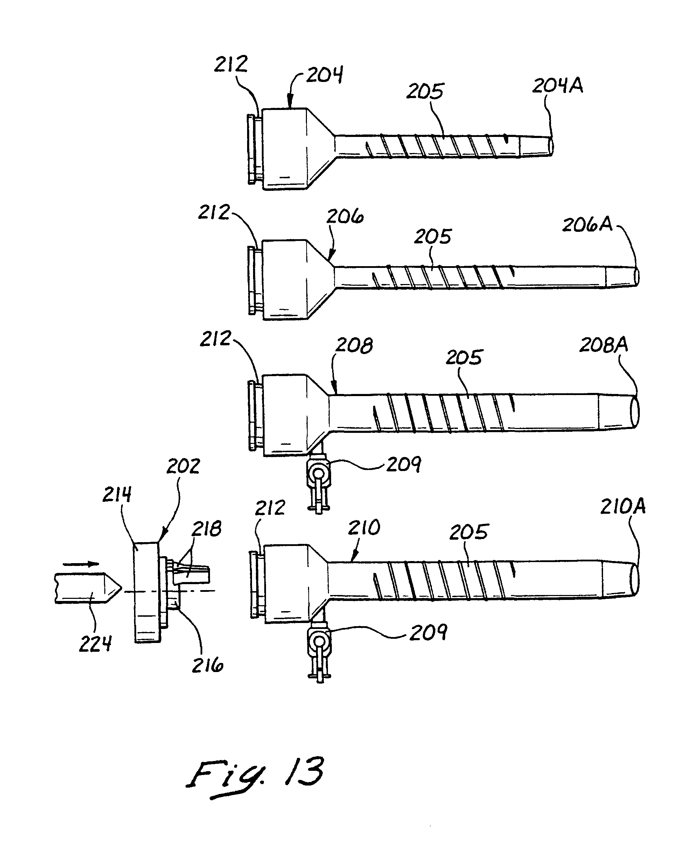

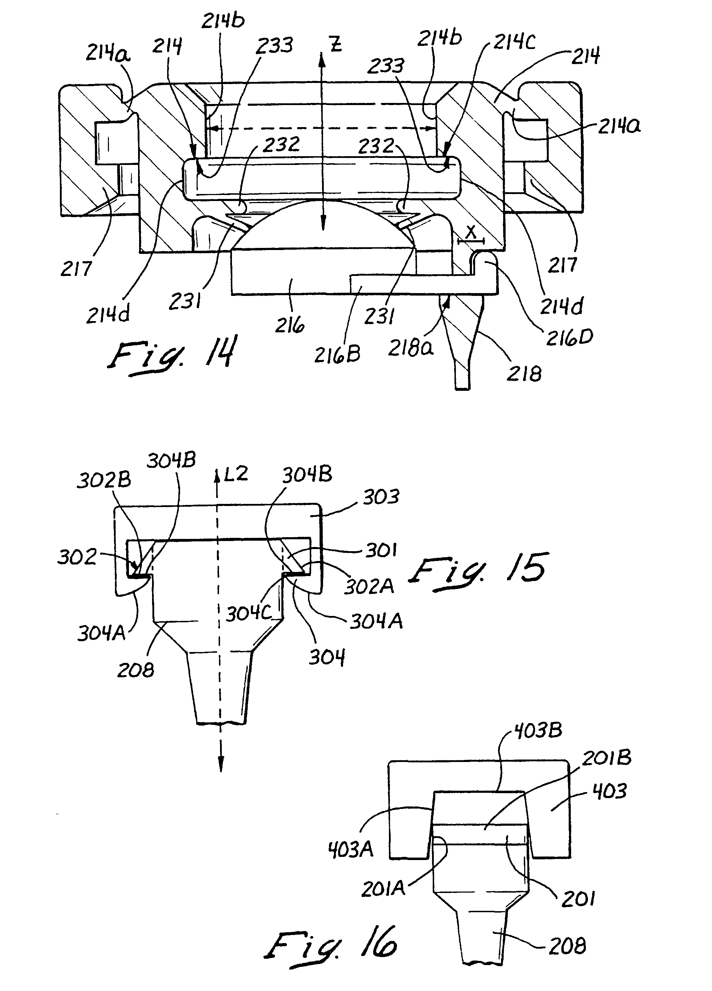

(seal not according to the invention) will be described with reference to Figures 8-9. Other (seals and structures, not falling under the invention) will be described with reference to Figs. 10-16.

The universal seal assembly of Fig. 3 comprises universal seal molding 21, base 88, and cap 33 (both typically formed of rigid plastic), and stabilizing ring halves 44A and 44B. Molding 21 comprises generally conical instrument seal 23 (seal 23 is the central portion of molding 21 which includes ridges 26 and bumper 25, and is sometimes referred to herein as the "instrument guide" of universal seal molding 21), stabilizing ring anchor 30 surrounding conical seal 23, corrugated annular portion 28 surrounding anchor 30, and anchoring ring 31 surrounding corrugated portion 28. Ring halves 44A and 44B are snapped together to enclose ring anchor 30 of seal 23, and base 88 and cap 33 are assembled together to define a rigid housing surrounding molding 21 and ring halves 44A and 44B. Cap 33 has an orifice 33A through its center, through which an instrument can be inserted. Conical seal 23 has an orifice 27 (also referred to as an instrument port) through its center, and functions to guide the tip of an instrument both radially inward toward orifice 27 and in the distal direction (downward along longitudinal axis L1 of Fig. 3) toward orifice 27.

Cap 33 and base 88 can be assembled together by snap fitting a flange of cap 33 into a peripheral groove around base 88 as shown. Elastomeric anchoring ring 31 of molding 21 is fixedly held between cap 33 and base 88 (when cap 33 and base 88 are assembled together with projection 33B of cap 33 locked in groove 38B of base 88 as shown) so as to form a fluid seal (i.e., a gas-tight and/or liquid-tight seal) between ring 31 and each of cap 33 and base 88.

A stabilizing ring for molding 21 is formed by stabilizing ring halves 44A and 44B. Each stabilizing ring half is an annular plastic ring half molding. Several (e.g., eight) equally-spaced pin holes 22 are formed through stabilizing ring anchor 30 of molding 21 (as shown in Fig. 4). Ring halves 44A and 44B are attached to molding 21 by inserting the pins of ring halves 44A through pin holes 22 in molding 21, and pressing the pins into corresponding pin holes in ring halves 44B.

Base 88 and cap 33 are shaped and sized so that when assembled together as shown, they allow assembled ring halves 44A and 44B to translate laterally on ledge 39 of base 88 (relative to fixed anchor portion 31) thereby carrying stabilizing ring anchor 30 and instrument seal 23 laterally relative to anchor portion 31, and accommodate bending and/or buckling motion of molding 21's corrugated zone 28 while anchor 30 and seal 23 so translate.

Flange 62 extends out from the distal end of base 88 for use in attaching the universal seal assembly to valve door assembly 202. Flange 62 is shaped to hook into the outer elastomeric sidewall of valve door assembly 202 to attach the universal seal assembly to valve door assembly 202 (preventing the universal seal assembly from sliding off assembly 202). Flange 62 has a barbed shape, with a distal surface 62B and a proximal surface 62A which meet at a circular edge 62C. Distal surface 62B of flange 62 is preferably ramp-shaped, and slopes both distally (i.e., longitudinally with increasing separation from longitudinal axis L1 with increasing distance from the proximal end of the universal seal assembly) and laterally so that it guides and facilitates the mounting of the universal seal assembly to seal assembly 202 (i.e., by screwing the flange along a generally helical path on the elastomeric body of assembly 201 until the flange hooks into the elastomeric body). Flange 62's proximal surface 62B is oriented more perpendicularly with respect to longitudinal axis L1 (when the apparatus is assembled as shown in Fig. 3) so that flange 62 can hook into the outer elastomeric sidewall of valve door assembly 202, to prevent the universal seal assembly from sliding in the proximal direction away from assembly 202 (and to prevent the universal seal assembly from being detached from assembly 202 by being "unscrewed" along a path opposite to a generally helical path along which the universal seal assembly was screwed onto assembly 202). Edge 62C of flange 62 sinks into the outer peripheral surface of assembly 202 so that surfaces 62A and 62B form a fluid seal with assembly 202.

In variations on the Fig. 3 embodiment, barb-shaped flange 62 is replaced by a flange having a plurality of barbs or teeth which grip the outer elastomeric sidewall of valve door assembly 202 to attach the universal seal assembly to valve door assembly 202, thereby preventing the universal seal assembly from sliding off assembly 202 and preferably also providing a fluid seal between the flange and the outer sidewall of valve door assembly 202. Optionally, a locking ring is placed around the outer periphery of the flange (having barbs or teeth), and the locking ring is tightened to cause the flange to more tightly grip the outer sidewall of valve door assembly 202 (and preferably also to improve the fluid seal between the flange and the outer sidewall of the valve door assembly).

Cap 33 and base 88 (including flange 62) are preferably made of rigid plastic. Inner sidewall 38A of the distal portion of base 88 maintains the proper lateral positioning of the universal seal assembly relative to valve door assembly 202. Distal surface 39A of ledge 39 of base 88 maintains the proper proximal-to-distal positioning of the universal seal assembly and valve door assembly 202 (by limiting the advancement of the universal seal assembly distally relative to assembly 202).

Valve door assembly 202 includes a flapper valve fluid seal which comprises a trap door (not shown) mounted on elastomeric barbed extensions 218, and an elastomeric flange 217 which can removably snap onto the proximal end of reusable cannula 208. Preferably, cannula 208 has a circular groove around its proximal end, and flange 217 is shaped so that it can be snapped into (and removed from) the groove to removably attach valve door assembly 202 to cannula 208. Valve door assembly 202 can be implemented as shown in (and described below with respect to) Fig. 14, or as in any of the disposable seal assembly embodiments disclosed in U.S. Patent 5,820,606, issued. october 13, 1998.

Generally conical portion 23 of seal molding 21 of Figs. 3-6 has radially oriented ribs 26 around central instrument port 27. Preferably, each rib 26 protrudes from both the distal face and the proximal face of portion 23, has a round profile (as shown in Fig. 5), and extends radially from a point near to port 27 all the way to bumper 25 (or to a point very near to bumper 25). Molding 21 is formed (as a single piece, as a result of a single molding operation) so that corrugated portion 28 biases port 27 to be centered about the central longitudinal axis L1 of cap 33, and conical portion 23 points in the distal direction (as shown in Fig. 6, in which arrow D denotes the distal direction) when the universal seal assembly has been assembled. Port 27 has freedom to translate in a plane perpendicular to longitudinal axis L1 in response to radial displacement of the port's rim (by an instrument extending therethrough), since corrugated portion 28 is compliant in the radial direction and flexes in response to the force exerted on seal portion 23 by the instrument.

The radial orientation of ribs 26 of molding 21 (and the size and profile of each rib) is designed to reduce instrument drag on an instrument being inserted in the distal direction toward or through port 27 (even if the instrument's diameter is the largest in the range of diameters around which the universal seal assembly can form a fluid seal), reduce the risk that an instrument will tear seal 23, prevent circumferential propagation of tears around seal 23, and add a desired degree of radial stiffness to seal 23 without undesirably reducing the expandability of instrument port 27. By adding radial stiffness to seal 23, ribs 26 (which act as a skeleton for seal 23) reduce leakage between seal 23 and an instrument extending through instrument port 27 is reduced (since ribs 26 center the instrument port around the instrument each time the instrument is displaced laterally during surgery).

Instrument port 27 of the Fig. 3 embodiment (and the instrument port of each other embodiment of the invention) must have smaller diameter than the diameter of each instrument inserted therethrough, so that the instrument can deform the elastic material surrounding the port to form a fluid seal around the instrument. Thus, when seal 23 is to accommodate a instruments having any diameter in a range of diameters (e.g., the range from 4 mm to 12 mm), instrument port 27 must have smaller diameter than the smallest diameter in the range, so that the minimum-diameter instrument can deform the elastic material surrounding the port. Deforming the elastic material results in a radial force between the elastic material and the instrument. This holds the elastic material in contact with the instrument and maintains the fluid seal.

Seal portion 23 of molding 21 (between ribs 26, and at port 27) should be thin enough to allow port 27 to expand with sufficiently low resistance as an instrument is inserted therethrough (so that the contact pressure between the instrument and seal portion 23 is low enough to reduce drag to an acceptable level), and thick enough for seal portion 23 to be adequately resistant to penetration by a pronged or hooked instrument.

Each rib 26 should be narrow enough so that seal portion 23 is predominantly thinner than the thickness T of each rib 26 (indicated in Fig. 5), to allow port 27 to expand with sufficiently low resistance as an instrument is inserted therethrough (so that the contact pressure between the instrument and seal portion 23 is low enough to reduce drag to an acceptable level). Each rib 26 should be thick enough so that an instrument guided by seal 23 toward port 27 rides along the ribs 26 (and not along the seal surface between ribs 26), and each rib should be narrow enough to reduce (to an acceptably low value) the contact area between seal 23 and each instrument guided by seal 23 toward port 27. The resistance due to friction on each such instrument being guided ("instrument drag") decreases with decreasing contact area between the seal and the instrument. Each rib 26 is preferably wide enough to define a gap between each pair of adjacent ribs 26 that is sufficiently narrow to reduce (to an acceptably low level) the probability that a sharp portion of an instrument will enter the gap and penetrate a thin portion of seal 23 between adjacent ribs 26.

Ribs 26 add robustness to instrument seal 23 in two ways. First, because they are oriented radially, they prevent circumferential propagation of tears around seal 23's conical surface. Second, they add bulk to the seal, thus reducing the risk of an instrument penetrating through the material comprising seal 23.

The preferred material comprising molding 21 (including all portions shown in Fig. 4) is medical grade silicone rubber with a durometer in the range from 40 through 70 Shore A Durometer. Similarly, the preferred material of other embodiments of the inventive universal seal molding (e.g., below-discussed moldings 1 and 11) is medical grade silicone rubber with a durometer in the range from 40 through 70 Shore A Durometer. Ribs 26 (or similarly functioning bumps, dimples, and other surface irregularities) on the surface of the instrument guide portion of each embodiment of the inventive universal seal molding are preferably molded with the rest of the molding as a single piece (as a result of a single molding operation), thus minimizing the cost of manufacturing each molding. Preferred implementations of the inventive universal seal assembly can be manufactured so inexpensively that each can be considered disposable (i.e., intended for use during a single medical procedure, after which it is discarded rather than being reused).

Bumper 25 extends around the periphery of seal portion 23 of molding 21, and protrudes in the proximal direction (toward the top of Fig. 3) when the universal seal assembly is assembled (as in Fig. 3) with generally conical seal portion 23 extending in the distal direction (and with ring halves 44A and 44B fixedly attached to and enclosing ring anchor 30 of seal 23). The function of bumper 25 is to cause seal portion 23 to rebound (relax) efficiently back to its conical configuration after portion 23 has inverted during withdrawal of an instrument in the proximal direction from instrument port 27. When an instrument has been inserted distally (in the direction of arrow D of Fig. 6) through port 27, seal portion 23 extends distally in its normal position (as shown in Figs. 3 and 6) and bumper 25 is separated from ring half 44A. Then, if the instrument is withdrawn proximally (in the direction of arrow P of Fig. 7), the force exerted by the instrument on port 27 may cause seal portion 23 to invert into the inverted position shown in Fig. 7, with bumper 25 compressed against ring half 44A and radially oriented ribs 26 pressed against bumper 25. Then, when the instrument has been totally withdrawn (out of engagement with seal 23), ribs 26 cease to press bumper 25 against ring half 44A and thus bumper 25 expands (as it relaxes back to its original configuration). As bumper 25 so expands, it urges seal portion 23 back to its original conical configuration (i.e., to the configuration shown in Figs. 3 and 6). Without such a bumper urging seal 23 back to its non-inverted configuration, there would be greater risk that seal 23 would remain in its inverted configuration after the instrument has been totally withdrawn. It is undesirable to allow the seal to remain in the inverted configuration since the seal is more vulnerable to penetration by pronged instruments in the inverted configuration.

Corrugated zone 28 of molding 21 can be designed in a conventional manner (e.g., as described above with reference to corrugated zone 52 of molding 40 of Figs. 1 and 2). For example, the lateral compliance of corrugated zone 28 is increased by molding it to have a series of substantially vertical elements interconnected by substantially semicircular sections as shown in Figs. 6 and 7. Lateral compliance is further increased by increasing the length of vertical elements. The material of corrugated zone 28 bends and/or buckles to accommodate lateral movement of instrument seal 23 (with stabilizing ring anchor 30 and ring halves 44A and 44B enclosing anchor 30) relative to fixed anchoring ring 31. implementations.

Next, an alternative embodiment of the universal seal molding (not belonging to the invention) will be described with reference to Figs. 8 and 9. Universal seal molding 1 of Figs. 8 and 9 has a generally conical portion 23 with radially oriented ribs 26 around central instrument port 27. In a variation on the Fig. 3 embodiment of the invention, molding 1 is substituted for seal molding 21 in the Fig. 3 assembly. Molding 1 of Figs. 8 and 9 differs from molding 21 of Figs. 3-6 only in that it lacks bumper 25. Stabilizing ring anchor 30, corrugated annular portion 28 surrounding anchor 30, anchoring ring 31 surrounding corrugated portion 28, and the other elements present in both molding 1 and molding 21 are numbered identically in Figs. 8 and 4, and the description of these elements provided above (with reference to Figs. 3-6) will not be repeated with reference to Figs. 8 and 9. Molding 1 performs all the functions of above-described molding 21, except that since it12 lacks bumper 25, it is subject to an increased risk of undesirably remaining in an inverted configuration after being inverted by an instrument as the instrument withdraws in the proximal direction through molding 1's instrument port 27.

Other seal moldings (not belonging to the invention) will next be described with reference to Figs. 10-12. Universal seal molding 11 of Fig. 10 has a generally conical portion 23 with a pattern of radially arrayed concave (or convex) surface features 15. In some implementations of seal 11 (e.g. that of Fig. 11), each feature 15 is a dimple (a concave surface feature, which can have the profile of dimples 15A of Fig. 11). In other implementations of seal 11 (e.g. that of Fig. 12), each feature 15 is a bump (a convex surface feature, which can have the profile of bumps 15B of Fig. 12. In variations on the Fig. 3 embodiment of the invention, seal molding 11 (of Fig. 10, 11, or 12) is substituted for seal molding 21 in the Fig. 3 assembly. Molding 11 of Figs. 10-12 differs from molding 21 of Figs. 3-6 only in that it lacks bumper 25 and in that features 15 replace ribs 26. Stabilizing ring anchor 30, corrugated annular portion 28 surrounding anchor 30, anchoring ring 31 surrounding corrugated portion 28, and the other elements present in both molding 11 and molding 21 are numbered identically in Figs. 4 and 10-12, and the description of these elements provided above (with reference to Figs. 3-6) will not be repeated with reference to Figs. 10-12. Molding 11 performs all the functions of above-described molding 21, except that since it lacks bumper 25, it is subject to an increased risk of undesirably remaining in an inverted configuration after being inverted by an instrument as the instrument withdraws in the proximal direction through molding 11's instrument port 27. Radially arrayed surface features 15 reduce instrument drag (by reducing the contact area between the seal and an instrument being guided by the seal along the seal surface), but do not provide the other benefits provided by radially oriented ribs 26 of Figs. 4 and 8 (i.e., features 15 do not reduce the risk that an instrument will tear the seal as much as ribs 26 do, features 15 do not prevent circumferential propagation of tears around the seal's conical surface, and features do not add as much radial stiffness to the seal as do ribs 26).

In variations on the (seals) of Figs. 10-12. a pattern of concave or convex surface features are arranged on the generally conical surface of the seal molding, but these features are not radially arrayed.

Next, with reference to Figs. 13 and 14, we describe in greater detail an embodiment of valve door assembly 202 and cannula 208 of Fig. 3, and three other reusable cannulae (204, 205, and 210) usable with assembly 202. Figs. 13 and 14 correspond to figures set forth in above-referenced U.S. Patent 5,820,606.

As shown in Fig. 13, valve door assembly 202 can be removably snapped onto the proximal end of any of reusable cannulae 204, 206, 208, and 210. Each of cannulae 204, 206, 208, and 210 has a distal end (end 204A, 206A, 208A, and 210A, respectively) which is inserted within a patient during use, and a proximal end opposite the distal end. Each cannula has a central longitudinal channel extending through it, and any of a variety of instruments (such as instrument 224) can be positioned in the channel while the cannula is inserted into the patient (or after the cannula has been inserted into the patient). More specifically, the distal portion of each cannula is a rigid, generally cylindrical tube whose bore has a circular cross-section. The bore of the tube defines a channel for accommodating an instrument such as an endoscope. In variations on this design, the bore of the cannula's distal portion can have any cross-sectional shape, but is sized and shaped to receive a medical instrument. Each cannula is preferably made of a rigid material such as metal or rigid plastic, and the outer wall of each cannula has a grip portion 205. Preferably, grip portion 205 is produced by scoring a pattern (e.g., a spiral pattern as shown in Fig. 13) into the outer metal surface of the cannula. Alternative embodiments of the cannulae have smooth outer walls.

The proximal end of each cannula is identically shaped and sized, so that seal assembly 220 can be snapped onto any of them. More specifically, the proximal end of each of cannulae 204, 206, 208, and 210 is generally cylindrical, and has an identical annular groove 212 around it. A flange portion (flange 217, shown in Figs. 3 and 14 but not in Fig. 13) of body portion 214 of seal assembly 202 can be snapped into groove 212 to removably attach seal assembly 202 onto any of the cannulae. Preferably, body 214 has a thin annular portion 214a (shown in Fig. 14) between flange 217 and the main portion of body 204. The thin portion 14a functions as a bellows allowing flange 217 to flex relative to the main portion of body 214, so that assembly 202 can be manipulated by a user without removing flange 217 from groove 212.

As shown in Fig. 13, stopcock 209 is mounted to an orifice which extends through each of cannulae 208 and 210 (e.g., orifice 208A of cannula 208, shown in Fig. 3). A source of insufflation gas can be connected to stopcock 209 at desired times during a medical procedure, and stopcock 209 can be opened (to allow gas to flow through the orifice into or out from cannula 208 or 210) or closed (to seal the orifice). Some embodiments of the reusable cannula have an orifice to which a stopcock can be mounted, while others (e.g., cannulae 204) do not have such an orifice.

Preferably, seal assembly 202 is molded from elastic material (such as medical grade silicon rubber or another medical grade elastomer), except for its rigid trap door 216 (which can be molded from hard plastic such as polycarbonate material). Thus, the seal assembly has a simple design consisting of two components only. In alternative embodiments, the trap door is made of non-rigid material. In some such alternative embodiments, the valve door assembly is molded as a single piece of elastomeric material (including an elastomeric trap door portion). Cannulae 204, 206, 208, and 210 are preferably made of rigid such as stainless steel (or another metal) or rigid plastic.

Two flexible extension portions 218 protrude out from body portion 214 of seal assembly 202, and trap door 216 is mounted on members 218. Preferably, each extension portion 218 has a barb 218a for retaining the door against body portion 214 after the extension portion's head (which preferably has a generally triangular cross-section) and the barb have been inserted through a slot through the door, and each extension portion 218 also has a flexible shaft connecting the head to body portion 214.

Body portion 214 has a trap door seat portion (flange 231 shown in Fig. 14) which surrounds the distal end of the central channel which extends through body portion 214. The central channel extends along central longitudinal axis Z of seal assembly 202 (shown in Fig. 14), and preferably has rotational symmetry about axis Z.

Trap door 216 is biased by barbed portions 218 so that it normally rests in a closed position against the trap door seat, so that the closed trap door 216 prevents fluid from flowing through the central channel. Trap door 216, barbed portions 218, and the trap door seat portion comprise a flapper valve, which functions as follows. Barbed portions 218 are flexible so that when seal assembly 202 has been snapped onto a cannula, and an instrument (e.g., instrument 224 of Fig. 13) is inserted through the central channel through body portion 214 (and through the trap door seat and into the channel extending through the cannula), the instrument displaces trap door 216 away from the trap door seat (thereby bending barbed portions 218) and continues into the cannula's channel (until the distal end of the instrument passes entirely through the channel and out the distal end of the cannula). Then, when the instrument is withdrawn from the cannula and seal 202, barbed portions 218 relax, thus urging trap door 216 back into its normal position preventing fluid flow past the trap door seat.

Preferably also, body 214 includes an instrument seal portion (flanges 231 and 232) which prevents fluid flow past an instrument which has been inserted through the central channel of seal assembly 202. Typically, such instrument seal portion is a simple flange (or double flange) which extends into the central channel through seal assembly 202. Because such a flange (or double flange) has a fixed diameter, it will not provide a good seal around instruments having diameter less than a particular value. Thus, the universal seal assembly of the invention (or an adaptor seal assembly) may be used with seal assembly 202, to adapt assembly 202 for use with a smaller diameter instrument. For example, when seal assembly 202 is snapped onto the end of large diameter cannula 210, relatively large diameter instrument 24 can be inserted through body 214 of assembly 202, and the instrument seal within body 214 provides a good fluid seal around instrument 24. If the user desires to insert an instrument having substantially smaller diameter than instrument 24 into cannula 210, the user snaps the inventive universal seal assembly (e.g., that of Fig. 3) onto body 214 (so that the central channels through body 214 and the universal seal assembly are aligned) and then inserts the relatively small diameter instrument through the universal seal assembly. When the relatively small diameter instrument is so inserted through the universal seal assembly into cannula 210, the instrument seal within adaptor 20 (or universal seal assembly) provides a good fluid seal around the relatively small diameter instrument, and the relatively small diameter instrument displaces door 216 of the flapper valve within body 214.

It is contemplated that any of a variety of variations on valve door assembly 202 can be used interchangeably with any of the cannulae. Some of such variations are valve door assemblies, each having at least one instrument seal (useful with instruments having a particular outer diameter or range of outer diameters) and a flapper valve. Others of such variations are disposable seal assemblies, each having at least one instrument seal (useful with instruments having a particular outer diameter or range of outer diameters) and a valve which provides a fluid seal when no instrument extends through the assembly.

In one embodiment, each of short cannula 204 and long cannula 206 has a channel of diameter slightly greater than 5 mm (for use with an instrument of 5 mm diameter), cannula 208 has a channel of diameter slightly greater than 10 mm (for use with an instrument of 10 mm diameter), and cannula 210 has a channel of diameter slightly greater than 12 mm (for use with an instrument of 12 mm diameter). For use with all such cannulae and instruments, the inventive universal seal assembly can include a seal molding (e.g., an appropriately sized implementation of molding 21 of Fig. 3) configured to provide a seal around an instrument having any diameter in the range from 4 mm to 12 mm, and body portion 214 of seal assembly 202 can include an instrument seal of a diameter which provides a seal around an instrument having any diameter in the range from 10 mm to 12 mm.

With reference again to Fig. 14, trap door 216 has a generally hemispherical portion which rests against flange 231 when the flapper valve is in its closed position shown in Fig. 14. Trap door 216 also has a flange 216B, and two slots extend through flange 216B. Trap door 216 is mounted to body 214 by inserting one barbed portion 218 through each slot through flange 216B until shoulder 218a (of each barbed portion) passes through the slot, and spacer portion 216D of door 216 abuts body 214. Thus, each barbed portion 218 is slightly stretched by the force exerted on shoulder 218a by door 216, and barbed portions 218 in turn exert a biasing force on door 216 tending to keep door 216 in the closed position against the trap door seat (as shown in Fig. 14). As the hemispherical portion of door 216 is displaced away from the trap door seat (by an instrument which pushes the hemispherical portion as the instrument translates through the central channel of body 214), barbed portions 218 will bend and also stretch, thus allowing door 216 and barbed portions 218 to pivot away from the instrument (together as a unit about spacer 216D).

The biasing torque exerted by barbed portions 218 on door 216 (tending to keep door 216 in the closed position) is T = 2PX, where P is the preload force exerted (toward the top of Fig. 14) on door 216 by each barbed portion 218, and X is the distance (shown in Fig. 14) between the center of spacer 216D and the central longitudinal axis of each barbed portion 218. Since X is greater than zero, door 216 is said to be mounted on an "over-centered" door hinge.

As door 216 is displaced (by an instrument) into its open position, barbed portions 218 will bend and stretch, and the distance between spacer 216D and the central axis of each barbed portion will be reduced to a value X' (where X' is less than X) as spacer 216D moves slightly to the left in Fig. 14). This effect tends to reduce the torque on door 216. The torque exerted by barbed portions 218 on door 216 (when door 16 is in its opened position) is T = 2P'X' + M, where M is the moment due to the bending of barbed portions 218, and P' is the increased preload force exerted on door 216 by each barbed portion 218 (P' is greater than P since barbed portions 218 become stretched, that is elongated, when door 216 is in its opened position). Door 216 and barbed portions 218 are designed so that no more than an acceptable maximum torque is exerted on door 216 by barbed portions 218, regardless of the angle by which door 216 is rotated (about its pivot point) from its closed orientation (e.g., so that the torque reduction caused by the reduced factor X' cancels a sufficient amount of the torque increase due to the parameters P' and M). If the torque exerted on door 216 (by barbed portions 218) is so controlled, the opened door 216 will exert no more than an acceptable amount of drag force on any instrument being translated through the central channel into (or out from) the cannula to which seal assembly 202 is connected.

In alternative (constructions) of the valve door assembly, only one barbed portion is used to attach the trap door to the seal assembly's body. In this case, the trap door would have only a single slot, rather than dual slots as described above. To ensure stable positioning of the door, such a single barbed portion would typically be wider than barbed portions 218 shown in Figs. 3 and 14, in the sense that it would extend farther around the periphery of the seal assembly's central channel than does either of barbed portions 218 (of Figs. 3 and 14).

Also, in alternative (constructions) of the valve door assembly, the trap door is attached to the seal assembly's flexible extensions (which are not necessarily barbed or arrow-shaped) other than by inserting the extensions through slots in the trap door. For example, the trap door could be attached to the extensions by glue or another adhesive, or the extensions could have one or more slots extending through them and the trap door could fit through such slot or slots.

Small diameter portion 214b of body 214 of seal assembly 202 (shown in Fig. 14) functions as a bumper to limit lateral motion of a large diameter instrument which occupies the central channel through assembly 202. If the central channel has a first radius R at a first end of body portion 214, bumper 214b has a smaller radius R', relative to the central longitudinal axis Z of the channel. Bumper 214b does not extend as far radially into the central channel as does flange 232 or 231 (so that it does not interfere with the sealing function of flange 231). Instead, bumper 214b functions to limit lateral movement of any large diameter instrument which extends through the channel. Preferably, bumper 214b is located low enough along the longitudinal axis of seal assembly 202 so that it rests against the cannula (when assembly 202 is snapped onto the end of the cannula), allowing loads from an instrument on bumper 214b to be borne by the inner wall of the cannula.

Circular flanges 231 and 232 of body portion 214 of seal assembly 202 function as an instrument seal. Circular flange 232 functions as a sacrificial seal in the following sense. Flange 232 comes into contact with an instrument being inserted downward along axis Z through the central channel through assembly 202 (into the cannula to which assembly 202 is mounted) before thin circular flange 231 does, and if flange 232 is displaced (or even cut) by a sharp instrument being so inserted, flange 231 will maintain a good fluid seal against the instrument (preventing fluid flow between flange 231 and the outer periphery of the instrument). Preferably, the radial distance between axis Z and flange 231 is less than the radial distance between axis Z and flange 232, and flange 231 is thinner than flange 232 as shown in Fig. 14. With this preferred design, if flange 232 is displaced by an instrument (or possibly even cut by a sharp instrument) being inserted downward along axis Z, the displaced portion of flange 232 (e.g., a displaced portion of flange 232 adjacent to a cut in flange 232) will fold downward, thereby causing a portion of flange 231 temporarily to open slightly (radially away from axis Z) so that flange 231 will avoid being cut by the instrument (as the instrument continues to advance past flange 232). After flange 231 opens temporarily in this manner, flange 231 (which is made of elastomeric material) will relax back to a position sealing the outer periphery of the instrument. Flange 231 is preferably oriented at an angle relative to sacrificial flange 232 as shown, to improve its ability to provide a good fluid seal with an instrument (but alternatively, flanges 231 and 232 are parallel or substantially parallel to each other). Flange 231 functions as an instrument seal as well as a trap door seat. If flange 232 is not cut by an instrument, both flanges 232 and 231 can function to seal around the outer periphery of an instrument which extends through them.

In variations on the above-described (constructions) of valve door assembly 202, the body portion of the valve door assembly includes an instrument seal which is separated along the central channel from a trap door seat. In such variations, the trap door seat can have a different shape than flange 31 shown in Fig. 14.

Alternative structures for use in mounting the inventive universal seal assembly to a cannula (or valve door assembly mounted on a cannula) will next be described with reference to Figs. 15-19.

In the Fig. 15 embodiment, valve door assembly 301 (rather than above-described valve door assembly 202) is removably snapped onto the proximal end of cannula 208. Assembly 301 can include a flapper valve fluid seal and an instrument seal (as does assembly 202), but assembly 301 differs from assembly 202 in that the elastomeric body of assembly 301 has a distal end portion 302 which defines a shoulder protruding radially outward from the side wall of cannula 208. Distal end portion 302 is typically made of elastomeric material (as is the entire body of assembly 301, in typical implementations) and distal end portion 302 is sufficiently flexible to allow mounting of assembly 303 onto assembly 301. The protruding shoulder defined by end portion 302 has a sloping proximal surface 302A and a distal surface 302B. Proximal surface 302A is oriented at an acute angle with respect to longitudinal axis L2 (the distance between surface 302A and axis L2 increases with increasing distance from assembly 301's proximal end) and distal surface 302B is preferably perpendicular to longitudinal axis L2 (when elements 208, 301, and 303 have been assembled together as shown in Fig. 15).

Universal seal assembly 303 has a housing made of rigid material (typically rigid plastic) which includes relatively large flange 304 (in place of relatively small flange 62 as in the Fig. 3 embodiment of the universal seal assembly), but assembly 303 preferably is otherwise identical to the universal seal assembly of Fig. 3. Flange 304 has a distal surface 304A and a proximal surface 304B which meet at circular edge 304C. Distal surface 304A is preferably oriented at substantially the same angle with respect to longitudinal axis L2 as is surface 302A (so that surface 302A does not impede the mounting of assembly 303 to assembly 301). Proximal surface 302B is preferably perpendicular to longitudinal axis L2 (so that surface 302B will engage surface 304B, when an effort is made to pull mounted assembly 303 away from assembly 301, thus impeding efforts to separate assembly 303 from assembly 301 after assembly 303 has been mounted to assembly 301 as shown in Fig. 15). Thus, assembly 303 can be easily mounted to assembly 301 by pushing assembly 303 distally onto assembly 301 (causing distal surface 304A of flange 304 to displace end portion 302 as surface 304A slides along surface 302A) until proximal surface 304B of flange 304 engages distal surface 302B (as shown in Fig. 15). In the assembled configuration shown in Fig. 15, assembly 303 is locked onto assembly 301 in the sense that relatively strong force in the proximal direction (much stronger than the distal force needed to lock the assembly 303 onto assembly 301) is needed to separate assembly 303 from assembly 301.

In the Fig. 16 (construction) (as in the Fig. 3 embodiment), valve door assembly 201 is removably snapped onto the proximal end of cannula 208. Universal seal assembly 403 has a housing made of rigid material (typically rigid plastic) which includes a tapered (frusto-conical) surface 403A configured for engagement with generally cylindrical outer sidewall 201A of seal assembly 201. Except for the shape of its rigid housing, seal assembly 403 is identical to the universal seal assembly of Fig. 3. Assembly 403 can be conveniently mounted to assembly 201 by pushing assembly 403 distally onto assembly 201, thereby causing tapered surface 403A (which preferably has a shallow taper) to compress the elastomeric body of assembly 201 until proximal end surface 201B of assembly 201 abuts distally facing surface 403B of assembly 403 (so that assembly 403 is locked onto assembly 201). The portions of surface 403A having smaller radius (those portions relatively near to surface 403B when assembly 403 is locked onto assembly 201) compress the body of assembly 201 more than do the portions of surface 403A having larger radius (those portions relatively far from surface 403B when assembly 403 is locked onto assembly 201). In the assembled configuration (with surface 403B abutting surface 201B), the body of assembly 201 is sufficiently compressed so that relatively strong force in the proximal direction (much stronger than the distal force needed to lock the assembly 403 onto assembly 201) is needed to separate assembly 403 from assembly 301.

Alternative assemblies employ alternative structures for mounting the universal seal assembly to a valve door assembly (removably snapped onto the proximal end of a cannula) and/or to the cannula (to which the valve door assembly is attached). For example, the universal seal assembly can include a rotating roller lock assembly (e.g., of the sprag clutch type) having a spring and configured for engagement with the generally cylindrical outer sidewall of the valve door assembly and/or the cylindrical outer sidewall of the cannula. For another example, the universal seal assembly can include a rubber friction cam assembly configured for engagement with the generally cylindrical outer sidewall of the valve door assembly and/or the cylindrical outer sidewall of the cannula.

The foregoing is merely illustrative and explanatory of preferred embodiments of the inventive methods and apparatus. Various changes in the component sizes and shapes, and other details of the embodiments described herein may be within the scope of the appended claims.

## Claims

1. A universal seal assembly for use with a cannula-seal system including a valve door assembly (202) having an elastomeric body, the universal seal assembly including:
a housing (33, 88) through which a channel extends, the housing (33, 88) having a mounting portion configured for attaching the housing (33, 88) to the elastomeric body of the valve door assembly (202); and
an elastomeric universal seal molding (21) having a periphery (31), a generally conical instrument guide (23) defining an instrument port (27), and a flexible corrugated portion (28) between the periphery (31) and the instrument guide (23), the molding (21) being mounted in the housing (33, 88) with the periphery (31) fixedly attached to the housing (33, 88) and the instrument guide (23) free to translate relative to the housing (33, 88) so that the instrument port (27) has variable position in the channel;
a ring (44A, 44B) attached to the molding (21) between the instrument guide (23) and the corrugated portion (28), the ring (44A, 44B) being configured to constrain motion of the instrument guide (23) in a direction perpendicular to the axis of the channel **characterised in that**
the molding (21) has a portion (25) which defines a bumper extending around the instrument guide (23) and protrudes toward the ring from the instrument guide, the bumper being sized and shaped to cause the universal seal molding to rebound efficiently off the ring (44A, 44B) to its original conical configuration after being inverted in response to exertion of force thereon.

2. An assembly according to claim 1, **characterised in that** the conical instrument guide (23) is provided with a plurality of radially extending, circumferentially spaced ribs (26) and **in that** the ribs (26) are configured to press against the bumper (25) when the seal is in an inverted configuration.

3. An assembly or an apparatus according to Claim 1 or to Claim 2, **characterised in that** the molding is made of medical grade silicone rubber having a durometer in the range from 40 through 70 Shore A Durometer.

4. An assembly according to any one of Claims 1 to 3, **characterised in that** the mounting portion is a barbed flange (62) protruding from the housing (33, 88), and is shaped to allow the housing (33, 88) to be moved along a first path relative to the elastomeric body (202) to mount the housing (33, 88) to the elastomeric body (202), and is shaped to hook into the elastomeric body (202) when moved opposite to the first path thereby impeding separation of the universal seal assembly from the valve door assembly (202) after the housing (33, 88) has been mounted to the elastomeric body (202).

5. An assembly according to any one of Claims 1 to 4, **characterised in that** the elastomeric body of the valve door assembly (202) has a generally cylindrical outer sidewall, the mounting portion is a tapered surface of the housing (33, 88), and the housing (33, 88) also has a distally facing surface, wherein the tapered surface is configured to surround and compress at least a portion of the elastomeric body (202) when the universal seal assembly is mounted to the valve door assembly (202) with the distally facing surface engaged with the valve door assembly (202).

6. An assembly according to Claim 4, **characterised in that** the housing (33, 88) is a rigid plastics housing, and the barbed flange (62) is a portion of the rigid plastics housing.

7. An assembly according to Claim 2, **characterised in that** the universal seal molding (21) is a single molded piece of elastomeric material and wherein the ribs (26) are configured to impart a desired degree of radial stiffness to the instrument guide without undesirably reducing expandability of the port.

## Patentansprüche

1. Universelle Abdichtungsbaugruppe zur Verwendung mit einem Kanülenabdichtungssystem einschließlich einer Ventiltürbaugruppe (202) mit einem elastomeren Körper, wobei die universelle Abdichtungsbaugruppe einschließt:
ein Gehäuse (33, 88) durch das sich ein Kanal erstreckt, wobei das Gehäuse (33, 88) ein Befestigungsteilstück aufweist, das zum Anbringen des Gehäuses (33, 88) an den elastomeren Körper der Ventiltürbaugruppe (202) konfiguriert ist; und
ein elastomeres, universelles Abdichtungsformteil (21) mit einer Peripherie (31), eine allgemein kegelförmige Instrumentenführung (23), die eine Instrumenten-Durchlassöffnung (27) definiert, und ein flexibles gewelltes Teilstück (28) zwischen der Peripherie (31) und der Instrumentenführung (23), wobei das im Gehäuse (33, 88) montierte Formteil (21) mit der Peripherie (31) starr am Gehäuse (33, 88) befestigt ist und sich die Instrumentenführung (23) relativ zum Gehäuse (33, 88) frei verschieben lässt, so dass die Instrumenten-Durchlassöffnung (27) variable Position im Kanal hat;
einen Ring (44A, 44B), der zwischen der Instrumentenführung (23) und dem gewellten Teilstück (28) am Formteil (21) befestigt ist, wobei der Ring (44A, 44B) konfiguriert ist die Bewegung der Instrumentenführung (23) in einer zur Achse des Kanals senkrechten Richtung zu beschränken, **dadurch gekennzeichnet, dass** das Formteil (21) ein Teilstück (25) aufweist, das einen Puffer definiert, der sich um die Instrumentenführung (23) erstreckt und ab der Instrumentenführung in Richtung des Rings hervorsteht, wobei der Puffer so bemessen und geformt ist, dasst er das wirksame Zurückprallen des universellen Abdichtungsformteils vom Ring (44A, 44B) in seine ursprüngliche kegelförmige Konfiguration bewirkt, nach dem er als Reaktion auf eine darauf ausgeübte Kraft invertiert wurde.

2. Baugruppe nach Anspruch 1, **dadurch gekennzeichnet, dass** die einpferchende Instrumentenführung (23) mit einer Vielheit sich radial erstreckender, am Umfang mit Abstand angeordneter Rippen (26) versehen ist und dadurch, dass die Rippen (26) konfiguriert sind gegen den Puffer (25) zu drücken, wenn sich die Abdichtung in einer invertierten Konfiguration befindet.

3. Baugruppe oder Vorrichtung nach Anspruch 1 oder nach Anspruch 2, **dadurch gekennzeichnet, dass** das Formteil aus Silikongummi medizinischer Qualität hergestellt ist und einen Härtemesser im Bereich von 40 bis zu 70 Shore A Härtemesser aufweist.

4. Baugruppe nach einem beliebigen der Ansprüche 1 bis 3, **dadurch gekennzeichnet, dass** das Befestigungsteilstück ein Flansch (62) von Widerhakenform ist, der aus dem Gehäuse (33, 88) hervorsteht, und so gestaltet ist, dass das Gehäuse (33, 88) entlang einer ersten Bahn relativ zum elastomeren Körper (202) bewegt werden kann, und gestaltet ist sich in den elastomeren Körper (202) einzuhaken, wenn er entgegengesetzt zur ersten Bahn bewegt wird und dadurch Trennen der universellen Abdichtungsbaugruppe von der Ventiltürbaugruppe (202) behindert nach dem das Gehäuse (33, 88) an den elastomeren Körper (202) montiert worden ist.

5. Baugruppe nach einem beliebigen der Ansprüche 1 bis 4, **dadurch gekennzeichnet, dass** der elastomere Körper der Ventiltürbaugruppe (202) eine allgemein zylindrische äußere Seitenwand aufweist, das Befestigungsteilstück eine konisch zulaufende Fläche des Gehäuses (33, 88) ist, und das Gehäuse (33, 88) außerdem eine distal gegenüberliegende Fläche aufweist, wobei die konisch zulaufende Fläche konfiguriert ist wenigstens ein Teilstück des elastomeren Körpers (202) zu umgeben und zusammenzudrücken, wenn die universelle Abdichtungsbaugruppe an die Ventiltürbaugruppe (202) montiert ist, wobei die distal gegenüberliegende Fläche mit der Ventiltürbaugruppe (202) in Eingriff steht.

6. Baugruppe nach Anspruch 4, **dadurch gekennzeichnet, dass** das Gehäuse (33, 88) ein starres Kunststoffgehäuse ist, und der Flansch (62) von Widerhakenform ein Teilstück des starren Kunststoffgehäuses ist.

7. Baugruppe nach Anspruch 2, **dadurch gekennzeichnet, dass** das universelle Abdichtungsformteil (21) ein einteilig geformtes Stück elastomeren Materials ist und wobei die Rippen (26) konfiguriert sind der Instrumentenführung einen gewünschten Grad radialer Steifheit mitzuteilen, ohne das Dehnvermögen der Durchiassöffnung unerwünscht zu reduzieren.

## Revendications

1. Un ensemble de joint d'étanchéité universel pour utilisation avec un système de joint à canule comprenant un ensemble de porte à soupape (202) ayant un corps en élastomère, l'ensemble de joint universel comprenant ;
un logement (33, 88) par lequel passe un canal, le logement (33, 88) ayant une portion montante configurée pour attacher le logement (33, 88) au corps en élastomère de l'ensemble de la porte de soupape (202) ; et
un moulage (21) de joint universel en élastomère ayant un pourtour (31), un guide-instrument (23), conique d'une façon générale, définissant un point d'accès (27) pour l'instrument, et une portion flexible ondulée (28) entre le pourtour (31) et le guide-instrument (23), le moulage (21) étant monté dans le logement (33, 88) avec le pourtour (31) fixé au logement (33, 88) et le guide-instrument (23) étant libre pour effectuer la translation par rapport au logement (33, 88), de sorte que le point d'accès de l'instrument (27) a une position variable dans le canal ;
un anneau (44A, 44B) attaché au moulage (21) entre le guide d'instrument (23) et la portion ondulée (28), l'anneau étant configuré pour obliger le guide-instrument (23) à se déplacer dans une direction perpendiculaire à l'axe du canal, **caractérisé en ce que**
le moulage (21) a une portion (25) qui définit une butée passant autour du guide-instrument (23) et qui projette du guide-instrument vers l'anneau, la butée étant de taille et de forme pour faire rebondir de façon efficace le moulage du joint universel de l'anneau (4A, 44B) et le faire revenir à sa configuration conique originale après avoir été inverti en réponse à une pression exercée dessus.

2. Un ensemble selon la revendication 1, **caractérisé en ce que** le guide-instrument conique (23) est pourvu d'un nombre de côtes (26) partant radialement et espacées en circonférence et que ces côtes (26) sont configurées de façon à appuyer contre la butée (25) quand le joint se trouve en une configuration invertie.

3. Un ensemble ou an appareil selon la revendication 1 ou 2, **caractérisé en ce que** le moulage est fait de caoutchouc de silicone de grade médical ayant un duromètre d'une gamme allant de 40 à 70 dureté Shore A.

4. Un ensemble selon l'une quelconque des revendications 1 à 3, **caractérisé en ce que** la portion montante est une bride barbelée (62) projetant du logement (33, 88) et qu'elle est façonnée pour permettre au logement (33, 88) d'être déplacé le long d'un premier trajet par rapport au corps en élastomère (202) pour monter le logement (33, 88) vers le corps en élastomère (202), et qui est formée de façon à se cramponner sur le corps en élastomère (202) quand il est déplacé en face du premier trajet, empêchant ainsi l'ensemble de joint universel de se séparer de l'ensemble de porte de soupape (202) après que le logement (33, 88) a été monté jusqu'au corps élastomère (202).

5. Un ensemble selon l'une quelconque des revendications 1 à 4, **caractérisé en ce que** le corps en élastomère de l'ensemble de la porte de soupape (202) a une paroi latérale extérieure de forme généralement cylindrique, la portion montante est une surface fuselée du logement (33, 88) et le logement (33, 88) a aussi une surface au point le plus éloigné, dans laquelle la surface fuselée est configurée pour entourer et comprimer au moins une portion du corps en élastomère (202) quand l'ensemble de joint universel est monté jusqu'à l'ensemble de porte de soupape (202) avec la surface éloignée engagée avec l'ensemble de porte de soupape (202).

6. Un ensemble selon la revendication 4, **caractérisé en ce que** le logement (33, 88) est un logement en matière plastique rigide et la bride barbelée (62) est une portion du logement en matière plastique rigide.

7. Un ensemble selon la revendication 2, **caractérisé en ce que** le moulage de joint universel (21) est un moulage de matière élastomère en une seule pièce et dans laquelle les côtes (26) sont configurées pour donner un degré de raideur souhaitable au guide-instrument sans toutefois réduire indûment la capacité d'expansion du point d'accès.
